# EUROPEAN PATENT SPECIFICATION

(11) **EP 3 244 967 B1**
(45) Date of publication and mention of the grant of the patent: **19.12.2018**
(21) Application number: 16701398.6
(22) Date of filing: 14.01.2016
(51) Int. Cl.: A61N 1/372, A61M 25/06, A61B 17/34, A61N 1/375, A61N 1/04, A61N 1/05

(54) **INTERVENTIONAL MEDICAL TOOLS AND ASSEMBLIES**
INTERVENTIONELLE MEDIZINISCHE INSTRUMENTE UND ANORDNUNGEN
OUTILS ET ENSEMBLES MÉDICAUX D'INTERVENTION

(30) Priority: 16.01.2015 US 201514598346
(43) Date of publication of application: 22.11.2017
(73) Proprietor: Medtronic, Inc., Minneapolis, MN 55432 (US)
(72) Inventor: WOOD, Rónán, Minneapolis, MN 55432 (US); WARD, Sean, Minneapolis, MN 55432 (US); MCDONNELL, Paula, Minneapolis, MN 55432 (US); MCHUGH, Pat, Minneapolis, MN 55432 (US)
(74) Representative: Dehns
(86) International application number: PCT/US2016/013347
(87) International publication number: WO 2016/115308

(56) References cited:
- US-A1- 2007 088 418
- US-A1- 2012 095 539
- US-A1- 2014 018 818

## Description

### FIELD OF THE DISCLOSURE

The present disclosure pertains to interventional medical systems, and more particularly to delivery tools and related assemblies that are configured to facilitate percutaneous transvenous deployment of relatively compact implantable medical devices.

### BACKGROUND

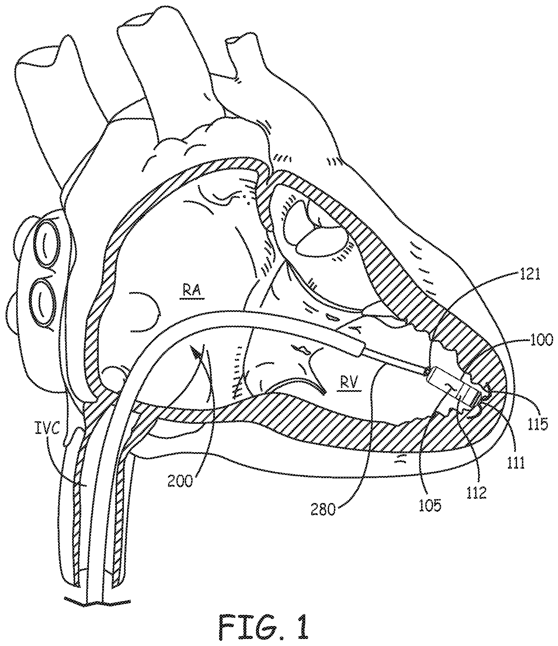

The traditional implantable cardiac pacemaker includes a pulse generator device to which one or more flexible elongate lead wires are coupled. The device is typically implanted in a subcutaneous pocket, remote from the heart, and each of the one or more lead wires extends therefrom to a corresponding electrode, coupled thereto and positioned at a pacing site, either endocardial or epicardial. Mechanical complications and/or MRI compatibility issues, which are sometimes associated with elongate lead wires and well known to those skilled in the art, have motivated the development of implantable cardiac pacing devices that are wholly contained within a relatively compact package for implant in close proximity to the pacing site, for example, within the right ventricle RV of the heart. With reference to Figure 1, such a device 100 is illustrated, wherein an hermetically sealed enclosure 105, preferably formed from a biocompatible and biostable metal such as titanium, contains a pulse generator, or an electronic controller and associated power source (not shown), to which at least one electrode 111 is coupled, for example, by a hermetic feedthrough assembly (not shown) like those known to those skilled in the art of implantable medical devices. Enclosure 105 may be overlaid with an insulative layer, for example, medical grade polyurethane, parylene, or silicone, and a portion of the insulation layer may be removed to form another electrode 112, for example, to provide bipolar pacing and sensing in conjunction with electrode 111.

Figure 1 illustrates device 100 having been deployed out from a distal portion of a delivery tool 200, which has been maneuvered up through the inferior vena cava IVC and into the right ventricle RV from the right atrium RA. Figure 1 further illustrates the deployed device 100 being fixed at an implant site by a fixation member 115 thereof, and an elongate tether 280, which extends within one or more lumens of delivery tool 200, being coupled to device 100, for example, looped through a tether attachment structure 121 formed at a proximal end of enclosure 105. Opposing ends of the looped tether 280 (not shown), which extend out from a proximal end of delivery tool 200, are accessible to an operator so that the operator may tug on tether 280 to test the fixation of device 100 at the implant site, and, if necessary, apply a greater force to tether 280 to remove device 100 from the implant site for repositioning at a more suitable site. But, if satisfied with the performance of device 100 at the illustrated implant site, the operator may release one end of the looped tether 280 and pull on the other end to withdraw tether 280 through delivery tool 150 and thereby remove tether 280 from attachment structure 122. Delivery tools are taught in US 2012/095539, US 2007/088418 and US 2014/018818.

### SUMMARY

Embodiments of the present invention facilitate increased efficiency of implantable medical device delivery tools, both in the assembly and construction thereof, and in their operation, as related to tether assemblies thereof. A tether assembly for a delivery tool, according to some embodiments, includes an elongate shaft and an attachment member coupled to a distal end of the shaft, wherein the attachment member includes an external thread and an end wall, the end wall extending orthogonally with respect to a direction of travel within a valley of the external thread to provide a hard stop for a threaded attachment bore of a medical device, when the bore is mated with the external thread. The tether assembly may further include a knob coupled to a proximal end of the shaft, wherein the aforementioned shaft translates torque, applied to the knob, from the knob to the attachment member in order to detach the tether assembly from the bore of the medical device, for example, after the device is implanted. According to some preferred embodiments, the elongate shaft of the tether assembly includes a cable formed from a plurality of wires wound together in a single direction about a longitudinal axis of the assembly, the direction being that in which the torque is applied to the knob to detach the tether assembly from the implanted device.

According to some embodiments, the above-described tether assembly extends within a longitudinally extending lumen defined by an elongate tube of an inner assembly of a delivery tool for an implantable medical device, such that the attachment member thereof is located in proximity to a distal member of the inner assembly, the distal member being coupled to a distal end of the tube and forming a distal opening of the lumen. In some delivery tool embodiments, a handle assembly of the delivery tool includes a valve member through which the shaft of the tether assembly extends, wherein a control member for the valve member may either rotate around the longitudinal axis of the tether assembly to open and close the valve member around the shaft of the tether assembly, or slide along the longitudinal axis to open and close the valve member around the shaft of the tether assembly.

### BRIEF DESCRIPTION OF THE DRAWINGS

The following drawings are illustrative of particular embodiments of the present disclosure and therefore do not limit the scope of the invention. The drawings are not to scale (unless so stated) and are intended for use in conjunction with the explanations in the following detailed description. Embodiments will hereinafter be described in conjunction with the appended drawings wherein like numerals denote like elements, and:
Figure 1 is a schematic showing an example of an implanted medical device for cardiac stimulation;
Figure 2A is a side view of a delivery tool for an implantable medical device, according to some embodiments;
Figures 2B-C are enlarged views of a distal end of the tool, with a partial cut-away section view, according to some embodiments;
Figure 3A is a plan view of an inner assembly of the delivery tool, according to some embodiments;
Figure 3B is a cross-section view through section line B-B of Figure 3A, according to some embodiments;
Figure 4A is a perspective view of the medical device positioned for mating with a tether assembly of the delivery tool, according to some embodiments;
Figure 4B is an enlarged perspective view of an attachment member of the tether assembly, according to some embodiments;
Figures 5A-B are schematics showing the delivery tool following deployment of the implantable medical device at an implant site, according to some embodiments; Figure 5C is an enlarged perspective view of a portion of a shaft of the tether assembly, according to some embodiments;
Figures 6A-B, 7, and 8 are plan views of various tether assembly embodiments; and
Figures 9A-B are perspective views of alternate embodiments of a handle assembly for the delivery tool.

### DETAILED DESCRIPTION

The following detailed description is exemplary in nature and is not intended to limit the scope, applicability, or configuration of the invention in any way. Rather, the following description provides practical examples, and those skilled in the art will recognize that some of the examples may have suitable alternatives.

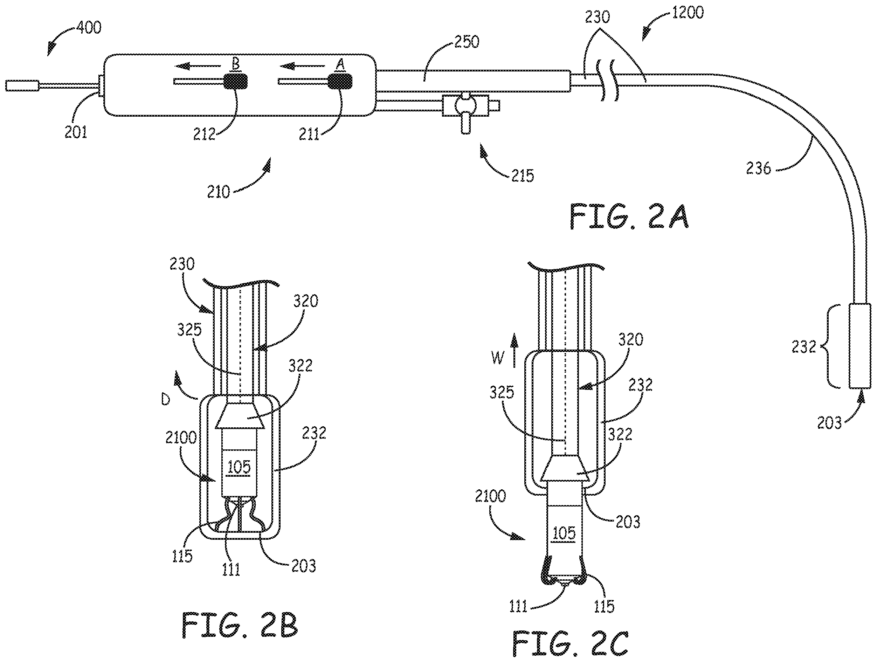

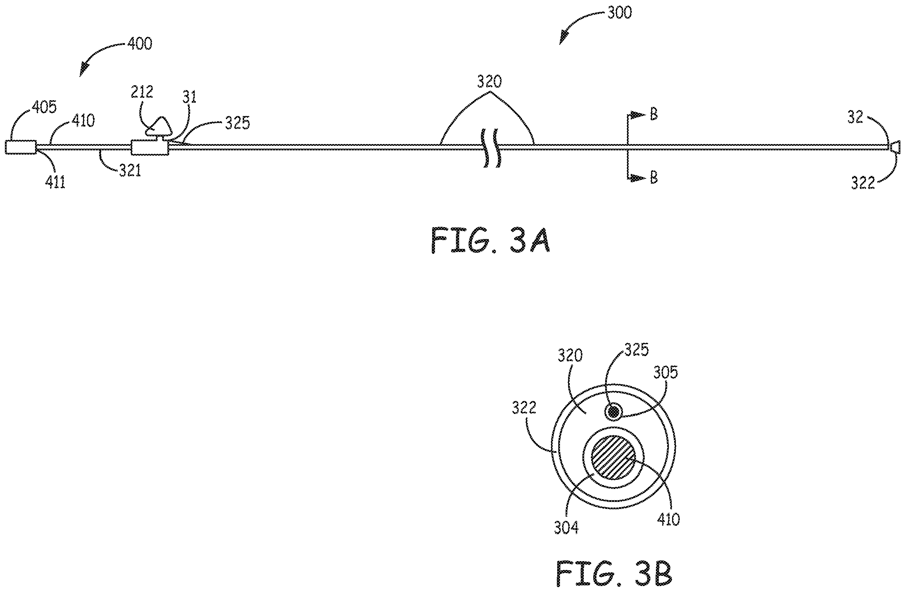

Figure 2A is a side view of a delivery tool 1200 for an implantable medical device, according to some embodiments; and Figures 2B-C are enlarged views of a distal end of tool 1200, with a partial cut-away section view, according to some embodiments. Figure 2A illustrates tool 1200 including a handle assembly 210 and an elongate deployment tube 230 surrounded by an outer, stabilizing sheath 250 in proximity to handle assembly 210. Figure 2A further illustrates deployment tube 230 including a distal-most portion 232, which is sized to enclose an implantable medical device 2100, for example, as shown in Figure 2B. Deployment tube 230 extends around another elongate tube 320, which can be seen in Figures 2B-C; tube 320 is part of an inner assembly 300, for example, as shown in Figures 3A-B. Figure 2A further illustrates handle assembly 210 including a flushing subassembly 215 to which a saline-filled syringe may be attached, for example, for flushing air from deployment tube 320 and/or from a lumen 304 (Figure 3B) of tube 320.

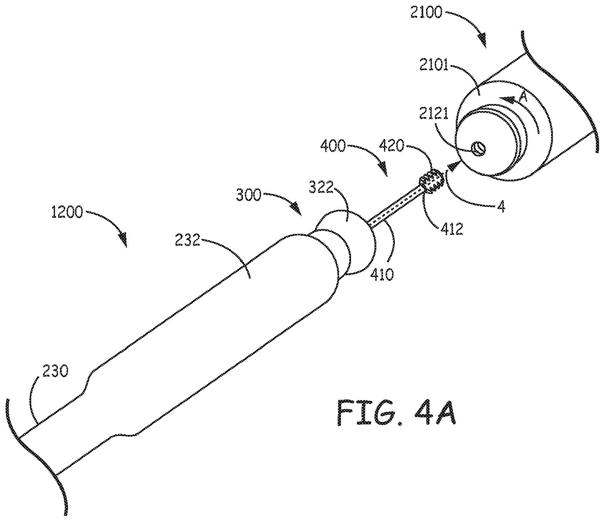

Figure 3A is a plan view inner assembly 300, according to some embodiments; and Figure 3B is a cross-section view through section line B-B of Figure 3A, according to some embodiments. Figures 2B-C and 3A-B illustrate inner assembly 300 further including a distal member 322, which is coupled to a distal end 32 of tube 320, and which is configured to conform to a proximal end 2101 of device 2100 (Figure 4A). With further reference to Figures 2B-C, distal-most portion 232 defines a distal opening 203 of deployment tube 230. According to the illustrated embodiment, deployment tube 230 is coupled to a control member 211 of handle assembly 210, which is operable, per arrow A, to retract, or withdraw tube 230 relative to stabilizing sheath 250 and inner assembly 300, per arrow W, so that device 2100 may be deployed through distal opening 203 for implant in a body of a patient, for example, within the right ventricle RV (Figure 1). Figures 2B-C and 3A-B further illustrate inner assembly 300 including a pull wire 325, which may extend within a longitudinally extending lumen 305 defined by tube 320, from a proximal end 31 thereof, which is coupled to another control member 212 of handle assembly 210, to a distal end that is anchored in proximity to distal end 32 of tube 320. Pull wire 325 may be a medical grade stainless steel wire with a fluoropolymer coating having a diameter of approximately 0.2286 mm (0.009 inch), and a diameter of lumen 305 may be approximately 03556 mm (0.014 inch). According to the illustrated embodiment, control member 212, when moved per arrow B, actuates pull wire 325 to bend inner assembly 300 and deployment tube 230, for example, to help navigate distal opening 203 of deployment tube 230 into proximity with a target implant site.

With further reference to Figures 2B-C, device 2100, like device 100 described above, includes hermetically sealed enclosure 105, which contains a pulse generator, electrode 111, and fixation member 115. The proximal end of device 2100, to which distal member 322 conforms, includes a tether attachment structure, which will be described below in conjunction with Figure 4A. With reference to Figures 2A and 3A-B, tool 1200 includes a tether assembly 400, which, according to Figures 3A-B, includes an elongate shaft 410 extending within lumen 304 defined by elongate tube 320 of inner assembly 300, and out through a proximal port 201 of handle assembly 210. A knob 405 of tether assembly 400 is shown coupled to a proximal end 411 of shaft 410, outside handle assembly 210, and, in Figure 4A, an attachment member 420 of tether assembly 400 is shown coupled to a distal end 412 of shaft 410. According to some embodiments, if attachment member 420 and knob 405 are each of a size that prevents passage thereof through lumen 304, knob 405 is coupled to shaft 410 by a removable attachment, for example, a spring-loaded clamping mechanism, so that knob 405 may be temporarily removed from tether assembly 400 so that shaft 410 can be back-loaded into lumen 304 by inserting proximal end 411 in through a distal opening thereof formed by distal member 322.

According to the illustrated embodiment, elongate tube 320 of inner assembly 300 is formed from bi-lumen tubing, for example, being extruded polyether block amide, polyurethane, or silicone rubber, or a composite thereof, and may include an overlay (not shown), for example, formed of braid-reinforced polyether block amide. Deployment tube 230 may be any suitable construction known in the art, for example, including varying durometers of polyether block amide to achieve a graduated flexibility and the necessary pushability and torque transfer that facilitates the maneuverability of tool 1200 to a target implant site. A detailed description of a suitable construction for deployment tube 230, and the assembly of such a deployment tube together with an inner assembly, similar to assembly 300, which is included in a co-pending and commonly assigned United States Patent Application having the Serial No. 14/039,937.

Figure 4A is a perspective view of a distal portion of delivery tool 1200 and a proximal end 2101 of medical device 2100, wherein each is positioned for loading device 2100 into distal-most portion 232 of deployment tube 230. Figure 4A illustrates deployment tube 230 withdrawn relative to inner assembly 300 so distal member 322 is exposed, and attachment member 420 tether assembly 400 is exposed for mating together with a threaded attachment bore 2121 of device 2100. According to the illustrated embodiment, upon initial engagement of attachment member 420 within bore 2121, device 2100 may be rotated around a longitudinal axis 4 of tether assembly 400, per arrow A, to mate bore 2121 with attachment member 420, after which proximal end 2101 of device 2100 may be drawn into distal member 322 of inner assembly 300, and deployment tube 230 may be advanced over distal member 322 and device 2100 to enclose both as illustrated in Figure 2B. According to an exemplary embodiment, enlarged distal-most portion 232 has an inner diameter of approximately 0.7 cm (-0.275 inch) and an outer diameter of approximately 0.8 cm (-0.3 inch), and may extend over a length of approximately 1.4 inch (-3.5 cm).

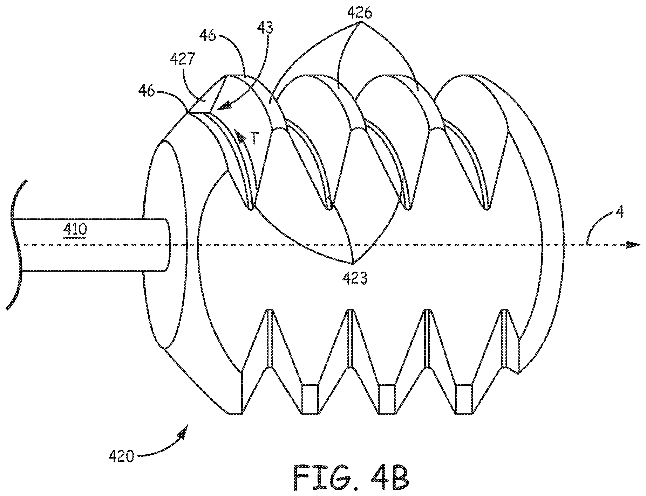

Figure 4B is an enlarged perspective view of tether assembly attachment member 420, according to some embodiments. Figure 4B illustrates attachment member 420 formed by an external thread, which is configured to mate with threaded attachment bore 2121 of medical device 2100, and which is defined by a peak 426 and a corresponding valley 423 that helically extend around longitudinal axis 4. Figure 4A further illustrates attachment member 420 including an end wall 427 that joins adjacent proximal sections 46 of peak 426 to terminate a proximal end 43 of valley 423. According to the illustrated embodiment, end wall 427 extends orthogonally with respect to a direction of travel T within valley 423 to provide a hard stop for threaded attachment bore 2121 of device 2100, when bore 2121 is mated with the external thread, as described above. Thus, end wall 427 prevents a wedging together of the mating threads when tightened together to the maximum depth, so that an undue amount of torque is not necessary to later detach tether assembly 400 from device 2100, as will be described below.

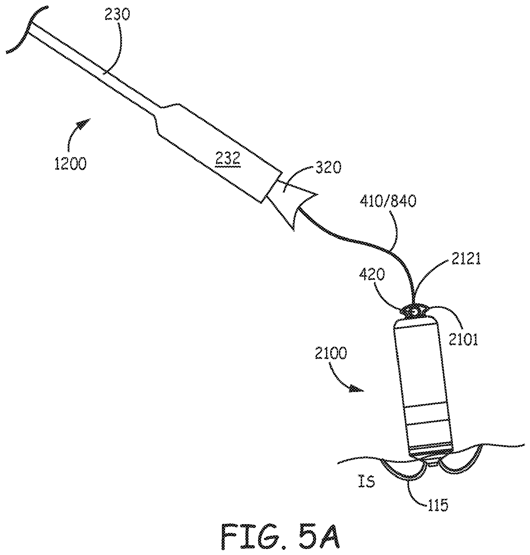

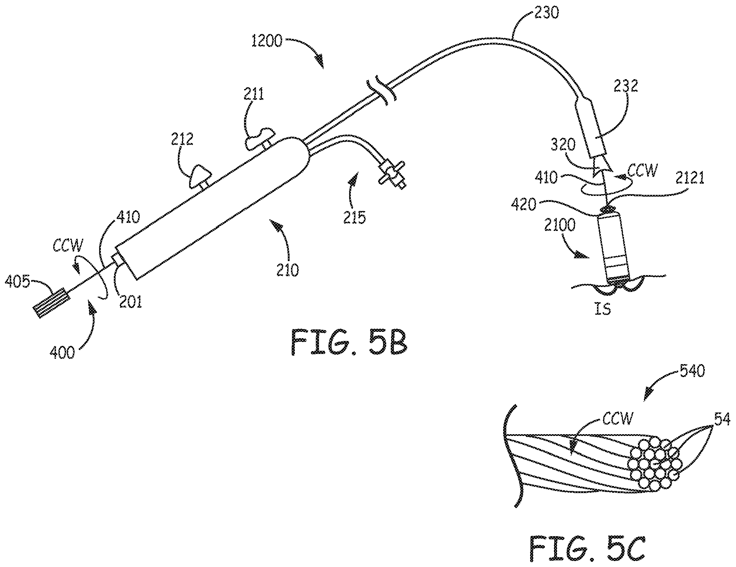

Figures 5A-B are schematics showing delivery tool 1200 following deployment of implantable medical device 2100 at an implant site IS, according to some embodiments. Figure 5A illustrates tool 1200 having been withdrawn, relative to tether assembly 400, which remains attached to device 2100, to expose a distal section of shaft 410 in proximity to attachment member 420, for example, while an operator evaluates performance of device 2100 at implant site IS. According to the illustrated embodiment, the distal section of tether assembly shaft 410 is sufficiently flexible so that the attachment thereof to device 2100 does not tug on implanted device 2100 to significantly impact the stability or orientation thereof, for example, as fixed in place via fixation member 115. Yet, with reference to Figure 5B, if the operator deems the performance of the implanted device 2100 acceptable, the flexibility of shaft 410 does not prevent tether assembly shaft 410 from transferring torque, per arrows CCW, from knob 405 to attachment member 420, so that the operator can detach tether assembly 400 from device 2100 by applying the torque to knob 405. As described above, because end wall 427 of attachment member 420 prevented wedging together of the mating threads of device 2100 and assembly 400, the torque that the operator applies to knob 405 for detachment readily rotates attachment member 420 relative to device attachment bore 2121, without being transferred through device fixation member 115 to compromise the implant. According to those embodiments in which knob 405 is too large to fit within lumen 304 of tube 320 (Figures 3A-B) and is coupled to shaft 410 by a removable attachment, the operator has an option to keep implanted device 2100 tethered while withdrawing delivery tool 1200 out from the patient's body and over shaft 410 after removing knob 405 therefrom. Alternately, in those embodiments where knob 405 and attachment member 420 are both too big to fit within lumen 304, when the operator has already detached tether assembly 400 from implanted device 2100 and removed delivery tool 1200 from the patient's body, the operator has an option to remove tether assembly 400 out from lumen 304, after knob 410 is removed from proximal end 411 of shaft 410, and then replace it with a snare assembly that can be employed to retrieve implanted device 2100, if necessary.

According to some embodiments, tether assembly shaft 410 is formed, at least in part, from a cable 540, for example, as illustrated in Figure 5C. Figure 5C is a perspective view of a portion of cable 540, which shows cable 540 being formed from a plurality of wires 54 wrapped, or wound together in a single direction, counter clockwise (CCW), which, being the same as the direction of the application torque, per arrows CCW of Figure 5B, maximizes the torque response of shaft 410 in detaching tether assembly 400 from device 2100. Attachment member 420 of tether assembly 400 may be joined to cable 540 by any suitable method known to those skilled in the art, but in some preferred embodiments, a weld joint is employed for the junction, to maintain a uniform diameter along a length of assembly 400. According to an exemplary embodiment, cable 540 has an outer diameter of approximately 0.254 mm (0.010 inch) and a 1x19 configuration known in the art, wherein nineteen medical grade stainless steel wires 54 each have an outer diameter of approximately 0.0381 mm (0.0015 inch). With further reference to Figure 5B, in conjunction with Figure 3B, when an entirety of tether assembly shaft 410 is formed from cable 540, according to some embodiments, inner assembly tube 320 is positioned relative to attachment member 420 to support shaft 410 for the above-described application of torque; thus when the outer diameter of shaft 410 is approximately 0.254 mm (0.010 inch), a diameter of lumen 304 of inner assembly tube 320 is approximately 0.3048 mm (0.012 inch). According to alternate embodiments, a tether assembly shaft including cable 540 is formed in two sections, proximal and distal, to maintain a balance between flexibility and torque transfer, without having to rely on lumen 304 for torque-transfer support; thus lumen 304 of inner assembly tube 320 may have a larger diameter, for example, being approximately 1.067 mm (0.042 inch), to accommodate other assemblies in lieu of tether assembly 400, for example, a snare assembly.

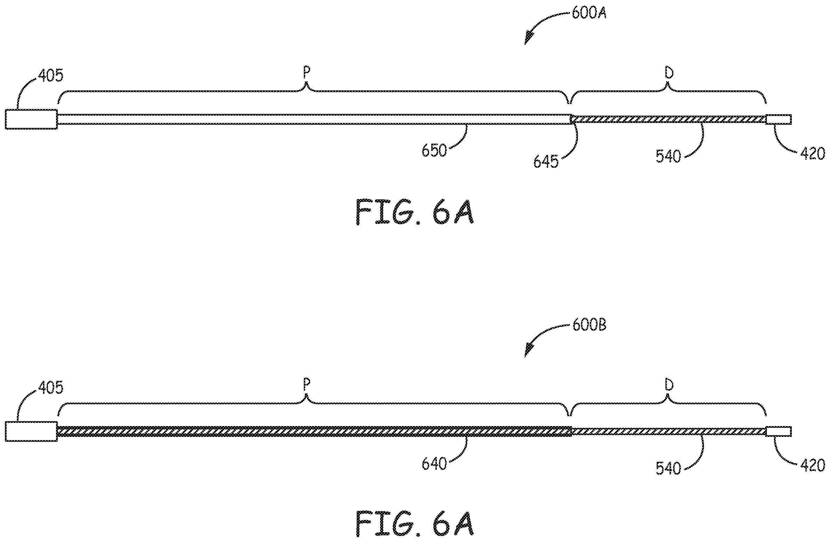

Figures 6A-B are plan views of tether assemblies 600A, 600B, which each include a proximal shaft section P and a distal shaft section D, wherein proximal shaft section P is stiffer than distal shaft section D. Figure 6A illustrates proximal shaft section P of assembly 600A being formed by a structure 650 that is separate from cable 540 of distal shaft section D. Structure 650 may be a medical grade stainless steel wire or hypo-tube that has an outer diameter of approximately 0.508 mm (0.020 inch). Figure 6A further illustrates a weld joint 645 attaching cable 540 to structure 650. Figure 6B illustrates proximal shaft section P of assembly 600B being formed by a polymer jacket 640, for example, a polyimide, overlaying a proximal length of cable 540. Jacket 640 may be formed around cable 540 by co-extrusion or a shrink fit. An outer diameter of proximal shaft section P of assembly 600B may be approximately 0.762 mm (0.030 inch). For each tether assembly 600A, 600B a length of proximal shaft section is between approximately 100 cm and 140 cm, and a length of distal shaft section D is between approximately 20 cm and 26 cm.

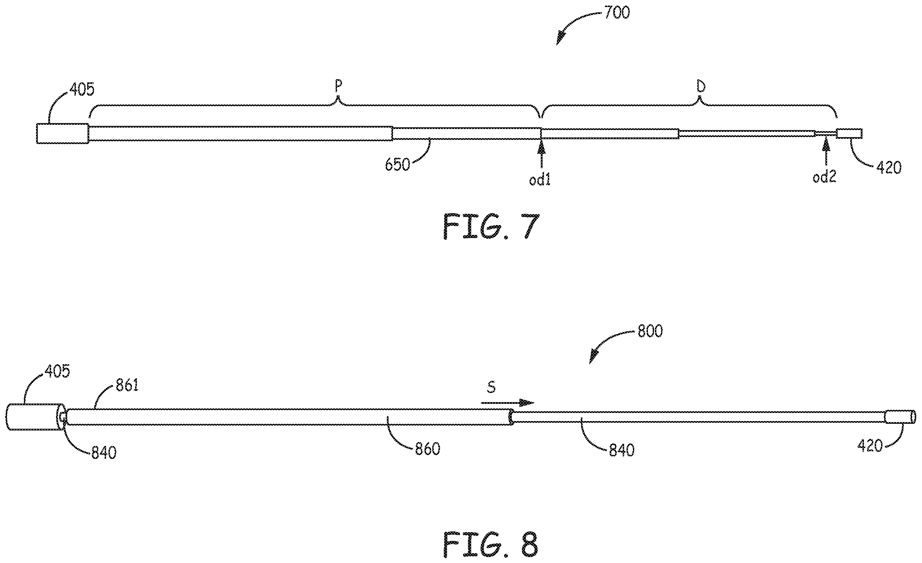

Figure 7 is a plan view of another tether assembly 700, according to some alternate embodiments, which also includes proximal shaft section P being stiffer than distal shaft section D. Figure 7 illustrates a tapered mandrel 750 (e.g., medical grade stainless steel wire) forming proximal shaft section P and distal shaft section D of assembly 700. Proximal shaft section P of assembly 700 may have a single diameter, for example, between approximately 0.254 mm (0.010 inch) and approximately 0.508 mm (0.020 inch), or may taper from a larger diameter (e.g., -0.508 mm (0.020 inch)), in proximity to knob 405, to a smaller diameter (e.g., -0.254 mm (0.010 inch)), in proximity to distal shaft section D; and distal shaft section D of assembly 700 tapers from a larger diameter, for example, between approximately 0.010 inch and approximately 0.254 mm (0.020 inch), to a smaller diameter, for example, approximately 0.0762 mm (0.003 inch). Attachment member 420 and knob 405 may be welded, crimped, or otherwise joined to opposing ends of tapered mandrel 750 according to methods known in the art.

Figure 8 is a plan view of yet another tether assembly 800, according to some additional embodiments. Figure 8 illustrates assembly 800 including a relatively flexible shaft 840 around which a stiffening tube 860 is slideably engaged. According to the illustrated embodiment, when tether assembly 800 is employed by delivery tool 1200, in lieu of tether assembly 400, a proximal end 861 of stiffening tube 860 is accessible outside handle assembly 210, proximal to proximal port 201 thereof (Figures 2A and 5B), so that the operator can slide tube 860 distally over flexible shaft 840, per arrow S, to provide torque-transfer support thereto, in proximity to attachment member 420. But, when an implantable medical device, for example, device 2100, which is attached to attachment member 420 of assembly 800, is deployed for implant, delivery tool 1200 is first withdrawn relative to flexible shaft 840 to expose a distal length thereof without the support of stiffening tube 860, for example, as illustrated in Figure 5A, for the evaluation of device performance, as described above. Then, if the device performance is deemed acceptable, the operator can advance stiffening tube 860 distally over the distal length of shaft 840, to provide the torque-transfer support thereto while applying the torque to knob 405 that detaches tether assembly 800 from implanted device 2100.

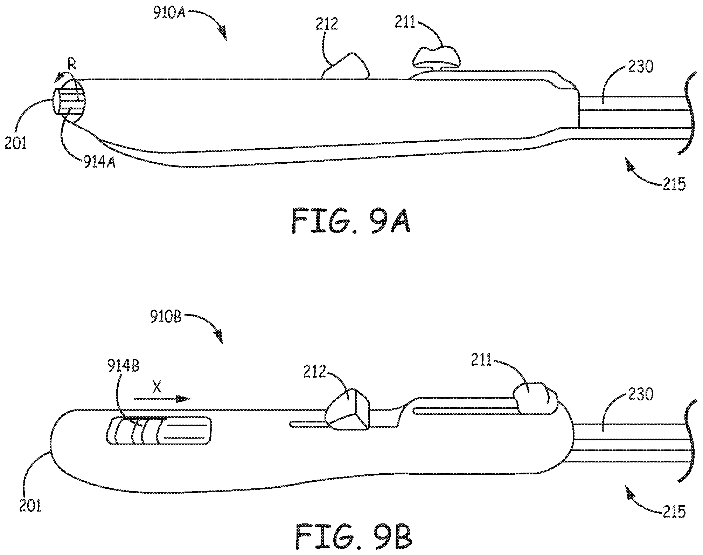

Figures 9A-B are perspective views of handle assemblies 910A, 910B, each of which may be incorporated by delivery tool 1200, in lieu handle assembly 210, according to alternate embodiments. Handle assemblies 910A, 910B are similar to handle assembly 210 in that each includes control members 211, 212, flushing subassembly 215, and proximal port 201. Figure 9A illustrates handle assembly 910A including a control member 914A for a valve member thereof, wherein the valve member is a Tuohy-bourst type known in the art so that control member 914A rotates, per arrow R, to open and close the valve member. Figure 9B illustrates handle assembly 910B including a control member 914B for a valve member thereof, wherein the valve member is a relatively soft tube that is collapsed by sliding control member 914B per arrow S. According to the illustrated embodiments, with reference back to Figures 3A-B, a proximal end 321 of inner assembly tube 320 is fixed within handle assembly 910A, 910B, in proximity to the valve member thereof, such that lumen 304 is in-line with the valve member, and shaft 410 of tether assembly 400 (alternately proximal shaft section P of any of tether assemblies 600A, 600B, 700, or stiffening tube 860 around flexible shaft 840 of tether assembly 800) extends through the valve member and out through proximal port 201. Thus, the valve member, of either handle assembly 910A, 910B, is operable, when closed, to seal around the shaft (or stiffening tube) of any of the above-described tether assemblies 400, 600A, 600B, 700, 800, and, when opened, to allow movement of the tether assembly therethrough, wherein control member 914A of handle assembly 910A rotates around the longitudinal axis of the tether assembly to open and close the corresponding valve member around the shaft thereof, and wherein control member 914B slides along the longitudinal axis to open and close the corresponding valve member around the shaft of the tether assembly.

In the foregoing detailed description, the invention has been described with reference to specific embodiments. However, it may be appreciated that various modifications and changes can be made without departing from the scope of the invention as set forth in the appended claims.

## Claims

1. A tether assembly for an implantable medical device, the medical device including an hermetic enclosure, a pulse generator contained within the enclosure, a fixation member coupled to a distal end of the enclosure, and a threaded attachment bore (1212) located in proximity to a proximal end of the enclosure, and the tether assembly comprising:
an elongate shaft (410) extending from a proximal end (411) thereof to a distal end (412) thereof;
an attachment member coupled to the distal end of the shaft, the attachment member including an external thread and an end wall, the external thread being defined by a helically extending peak (426) and a corresponding valley (423),
a knob (405) coupled to the proximal end of the shaft; and
wherein the shaft translates torque, applied to the knob, from the knob to the attachment member to detach the tether from the bore of the medical device; and **characterized by** the end wall (427) joining adjacent proximal sections of the peak to terminate a proximal end of the valley, the end wall extending orthogonally with respect to a direction of travel within the valley to provide a hard stop for the threaded attachment bore of the medical device, when the bore is mated with the external thread.

2. The assembly of claim 1, wherein the shaft comprises a cable (540) formed by a plurality of wires (54) wound together in a single direction, the direction being that in which the torque is applied to the knob of the tether assembly to detach the tether assembly from the bore of the medical device.

3. The assembly of any of claims 1 or 2, wherein:
the shaft includes a proximal section (P) and a distal section (D);
the proximal section extends from the proximal end of the shaft to the distal section, being formed by a single wire or a hypo-tube to which the cable is attached; and
the distal section extends from the proximal section to the distal end of the shaft, being formed by the cable.

4. The assembly of any of claims 1-3, wherein:
the cable extends along an entire length of the shaft; and
the shaft further comprises a polymer jacket extending around the cable only along a proximal length of the shaft.

5. The assembly of claim any of claims 1-4, wherein:
the shaft comprises a mandrel (750) and a stiffening tube (860) extending thereabout, the mandrel extending along an entire length of the shaft, and a length of the stiffening tube being less than that of the mandrel; and
the stiffening tube slides along the length of the mandrel between the proximal end and the distal end of the shaft.

6. The assembly of any of claims 1-5, wherein the shaft comprises a tapered mandrel (750) extending along an entire length of the shaft, the mandrel tapering from a first outer diameter along a proximal section thereof to a second outer diameter at the distal end of the shaft, the second diameter being smaller than the first diameter.

7. The assembly of claim 1, wherein the knob is coupled to the shaft by a removable attachment.

8. A delivery tool (1200) for an implantable medical device, the medical device including an hermetic enclosure (105), a pulse generator contained within the enclosure, a fixation member (115) coupled to a distal end of the enclosure, and a threaded attachment bore located in proximity to a proximal end of the enclosure, and the tool comprising:
a handle assembly (210) including a first control member (211), a second control member (212), a proximal port, and a valve member coupled to the second control member and being in-line with the proximal port;
an inner assembly (300) including an elongate tube (320) and a distal member (322), the tube including a proximal end (31) fixed within the handle assembly in proximity to the valve member, the tube defining a longitudinally extending lumen, the lumen being in-line with the valve member, and the distal member being coupled to a distal end (32) of the tube, the distal member defining a distal opening of the lumen and being configured to conform to the proximal end of the hermetic enclosure of the medical device;
an outer elongate deployment tube (230) extending around the tube of the inner assembly and being longitudinally moveable, via the first control member of the handle assembly, relative to the inner assembly, the deployment tube including a distal-most portion defining a distal opening (203) of the deployment tube, and being sized to enclose the medical device and the distal member of the inner assembly; and
a tether assembly (400) as claimed in any preceding claim.

9. The tool of claim 8, wherein the second control member (212) of the handle assembly rotates around the longitudinal axis to open and close the valve member around the shaft of the tether assembly.

10. The tool of any of claims 8 or 9, wherein the second control member (212) of the handle assembly slides along the longitudinal axis to open and close the valve member around the shaft of the tether assembly.

## Patentansprüche

1. Halterungsanordnung für eine implantierbare medizinische Vorrichtung, wobei die medizinische Vorrichtung ein hermetisches Gehäuse, einen in dem Gehäuse enthaltenen Impulsgenerator, ein an ein distales Ende des Gehäuses gekoppeltes Fixierelement und eine in der Nähe eines proximalen Endes des Gehäuses befindliche, mit Gewinde versehene Befestigungsbohrung (1212) beinhaltet und wobei die Halterungsanordnung Folgendes umfasst:
einen länglichen Schaft (410), der sich von einem proximalen Ende (411) davon zu einem distalen Ende (412) davon erstreckt;
ein an das distale Ende des Schafts gekoppeltes Befestigungselement, wobei das Befestigungselement ein Außengewinde und eine Endwand beinhaltet, wobei das Außengewinde durch eine sich schraubenförmig erstreckende Spitze (426) und ein entsprechendes Tal (423) definiert ist,
einen Knopf (405), der an das proximale Ende des Schafts gekoppelt ist; und
wobei der Schaft ein auf den Knopf ausgeübtes Drehmoment von dem Knopf auf das Befestigungselement überträgt, um die Halterung von der Bohrung der medizinischen Vorrichtung zu lösen; und **dadurch gekennzeichnet, dass** die Endwand (427) benachbarte proximale Abschnitte der Spitze verbindet, um ein proximales Ende des Tals zu begrenzen, wobei sich die Endwand orthogonal in Bezug auf eine Bewegungsrichtung innerhalb des Tals erstreckt, um einen harten Anschlag für die mit Gewinde versehene Befestigungsbohrung der medizinischen Vorrichtung bereitzustellen, wenn die Bohrung mit dem Außengewinde in Eingriff gebracht wird.

2. Anordnung nach Anspruch 1, wobei der Schaft ein Kabel (540) umfasst, das durch eine Vielzahl von Drähten (54) gebildet ist, die in einer einzigen Richtung zusammen gewickelt sind, wobei die Richtung diejenige ist, in der das Drehmoment auf den Knopf der Halterungsanordnung ausgeübt wird, um die Halterungsanordnung von der Bohrung der medizinischen Vorrichtung zu lösen.

3. Anordnung nach einem der Ansprüche 1 oder 2, wobei:
der Schaft einen proximalen Abschnitt (P) und einen distalen Abschnitt (D) beinhaltet;
sich der proximale Abschnitt von dem proximalen Ende des Schafts zu dem distalen Abschnitt erstreckt, wobei er durch einen einzelnen Draht oder eine Hyporöhre gebildet ist, an der das Kabel befestigt ist; und
sich der distale Abschnitt von dem proximalen Abschnitt zu dem distalen Ende des Schafts erstreckt, wobei er durch das Kabel gebildet ist.

4. Anordnung nach einem der Ansprüche 1-3, wobei:
sich das Kabel entlang einer gesamten Länge des Schafts erstreckt; und
der Schaft ferner einen Polymermantel umfasst, der sich nur entlang einer proximalen Länge des Schafts um das Kabel herum erstreckt.

5. Anordnung nach einem der Ansprüche 1-4, wobei:
der Schaft einen Dorn (750) und ein Versteifungsrohr (860) umfasst, das sich um diesen herum erstreckt, wobei sich der Dorn entlang einer gesamten Länge des Schaftes erstreckt, und eine Länge des Versteifungsrohrs kleiner als die des Dorns ist; und
das Versteifungsrohr entlang der Länge des Dorns zwischen dem proximalen Ende und dem distalen Ende des Schafts gleitet.

6. Anordnung nach einem der Ansprüche 1-5, wobei der Schaft einen sich verjüngenden Dorn (750) umfasst, der sich entlang einer gesamten Länge des Schaftes erstreckt, wobei sich der Dorn von einem ersten Außendurchmesser entlang eines proximalen Abschnitts davon zu einem zweiten Außendurchmesser am distalen Ende des Schafts erstreckt, wobei der zweite Durchmesser kleiner als der erste Durchmesser ist.

7. Anordnung nach Anspruch 1, wobei der Knopf durch eine entfernbare Befestigung an den Schaft gekoppelt ist.

8. Abgabeinstrument (1200) für eine implantierbare medizinische Vorrichtung, wobei die medizinische Vorrichtung ein hermetisches Gehäuse (105), einen in dem Gehäuse enthaltenen Impulsgenerator, ein an ein distales Ende des Gehäuses gekoppeltes Fixierelement (115) und eine in der Nähe eines proximalen Endes des Gehäuses befindliche, mit Gewinde versehene Befestigungsbohrung beinhaltet und wobei das Instrument Folgendes umfasst:
eine Griffanordnung (210) mit einem ersten Steuerelement (211), einem zweiten Steuerelement (212), einem proximalen Anschluss und einem Ventilelement, das an das zweite Steuerelement gekoppelt ist und an dem proximalen Anschluss ausgerichtet ist;
eine innere Anordnung (300) mit einem länglichen Rohr (320) und einem distalen Element (322), wobei das Rohr ein proximales Ende (31) beinhaltet, das in der Griffanordnung in der Nähe des Ventilelements fixiert ist, wobei das Rohr ein sich in Längsrichtung erstreckendes Lumen definiert, wobei das Lumen an dem Ventilelement ausgerichtet ist und das distale Element an ein distales Ende (32) des Rohrs gekoppelt ist, wobei das distale Element eine distale Öffnung des Lumens definiert und so konfiguriert ist, dass es dem proximalen Ende des hermetischen Gehäuses der medizinischen Vorrichtung entspricht;
ein äußeres längliches Einsatzrohr (230), das sich um das Rohr der inneren Anordnung herum erstreckt und in Längsrichtung über das erste Steuerelement der Griffanordnung relativ zu der inneren Anordnung bewegbar ist, wobei das Einsatzrohr einen am weitesten distal gelegenen Abschnitt beinhaltet, der eine distale Öffnung (203) des Einsatzrohrs definiert und so bemessen ist, dass er die medizinische Vorrichtung und das distale Element der inneren Anordnung umschließt; und
eine Halterungsanordnung (400) nach einem der vorhergehenden Ansprüche.

9. Instrument nach Anspruch 8, wobei sich das zweite Steuerelement (212) der Griffanordnung um die Längsachse dreht, um das Ventilelement um den Schaft der Halterungsordnung herum zu öffnen und zu schließen.

10. Instrument nach einem der Ansprüche 8 oder 9, wobei das zweite Steuerelement (212) der Griffanordnung entlang der Längsachse gleitet, um das Ventilelement um den Schaft der Halterungsanordnung herum zu öffnen und zu schließen.

## Revendications

1. Ensemble d'attache pour dispositif médical implantable, le dispositif médical comprenant une enceinte hermétique, un générateur d'impulsions contenu dans l'enceinte, un élément de fixation couplé à une extrémité distale de l'enceinte et un alésage de fixation fileté (1212) situé à proximité d'une extrémité proximale de l'enceinte, et l'ensemble d'attache comprenant :
un arbre allongé (410) s'étendant d'une extrémité proximale (411) de celui-ci à une extrémité distale (412) de celui-ci ;
un élément de fixation couplé à l'extrémité distale de l'arbre, l'élément de fixation comprenant un filetage externe et une paroi d'extrémité, le filetage externe étant défini par un sommet s'étendant en hélice (426) et une vallée correspondante (423),
un bouton (405) couplé à l'extrémité proximale de l'arbre ; et
dans lequel l'arbre traduit le couple, appliqué au bouton, du bouton à l'élément de fixation pour détacher l'attache de l'alésage du dispositif médical ; et **caractérisé en ce que** la paroi d'extrémité (427) joint des sections proximales adjacentes du sommet pour terminer une extrémité proximale de la vallée, la paroi d'extrémité s'étendant perpendiculairement par rapport à une direction de déplacement dans la vallée pour fournir une butée dure pour l'alésage de fixation fileté du dispositif médical, lorsque l'alésage est couplé avec le filetage externe.

2. Ensemble selon la revendication 1, dans lequel l'arbre comprend un câble (540) formé par une pluralité de fils (54) enroulés ensemble dans une direction unique, la direction étant celle dans laquelle le couple est appliqué sur le bouton de l'ensemble d'attache pour détacher l'ensemble d'attache de l'alésage du dispositif médical.

3. Ensemble selon l'une quelconque des revendications 1 ou 2, dans lequel :
l'arbre comprend une section proximale (P) et une section distale (D) ;
la section proximale s'étend de l'extrémité proximale de l'arbre à la partie distale,
étant formée par un seul fil ou un hypo-tube auquel le câble est attaché ; et
la section distale s'étend de la section proximale à l'extrémité distale de l'arbre, étant formée par le câble,

4. Ensemble selon l'une quelconque des revendications 1 à 3, dans lequel :
le câble s'étend sur toute la longueur de l'arbre ; et
l'arbre comprend en outre une gaine en polymère s'étendant autour du câble uniquement le long d'une longueur proximale de l'arbre.

5. Ensemble selon l'une quelconque des revendications 1 à 4, dans lequel :
l'arbre comprend un mandrin (750) et un tube raidisseur (860) s'étendant autour de celui-ci, le mandrin s'étendant sur toute la longueur de l'arbre, et une longueur du tube raidisseur étant inférieure à celle du mandrin ; et
le tube raidisseur coulisse sur toute la longueur du mandrin entre l'extrémité proximale et l'extrémité distale de l'arbre.

6. Ensemble selon l'une quelconque des revendications 1 à 5, dans lequel l'arbre comprend un mandrin conique (750) s'étendant sur toute la longueur de l'arbre, le mandrin se rétrécissant à partir d'un premier diamètre extérieur le long d'une section proximale de celui-ci à un second diamètre extérieur au niveau de l'extrémité distale de l'arbre, le second diamètre étant inférieur au premier diamètre.

7. Ensemble selon la revendication 1, dans lequel le bouton est couplé à l'arbre par une fixation amovible.

8. Outil de distribution (1200) pour un dispositif médical implantable, le dispositif médical comprenant une enceinte hermétique (105), un générateur d'impulsions contenu dans l'enceinte, un élément de fixation (115) couplé à une extrémité distale de l'enceinte et un alésage de fixation fileté situé à proximité d'une extrémité proximale de l'enceinte, et l'outil comprenant :
un ensemble de poignée (210) comprenant un premier élément de commande (211), un second élément de commande (212), un orifice proximal et un élément de soupape couplé au second élément de commande et étant aligné avec l'orifice proximal ;
un ensemble interne (300) comprenant un tube allongé (320) et un élément distal (322), le tube comprenant une extrémité proximale (31) fixée à l'intérieur de l'ensemble de poignée à proximité de l'élément de soupape, le tube définissant une lumière s'étendant longitudinalement, la lumière étant alignée avec l'élément de soupape, et l'élément distal étant couplé à une extrémité distale (32) du tube, l'élément distal définissant une ouverture distale de la lumière et étant conçu pour se conformer à l'extrémité proximale de l'enceinte hermétique du dispositif médical ;
un tube de déploiement allongé extérieur (230) s'étendant autour du tube de l'ensemble intérieur et pouvant être déplacé longitudinalement, via le premier élément de commande de l'ensemble de poignée, par rapport à l'ensemble interne, le tube de déploiement comprenant une partie la plus distale définissant une ouverture distale (203) du tube de déploiement, et étant dimensionné pour enfermer le dispositif médical et l'élément distal de l'ensemble interne ; et
un ensemble d'attache (400) selon l'une quelconque des revendications précédentes.

9. Outil selon la revendication 8, dans lequel le second élément de commande (212) de l'ensemble de poignée tourne autour de l'axe longitudinal pour ouvrir et fermer l'élément de soupape autour de l'arbre de l'ensemble d'attache.

10. Outil selon l'une quelconque des revendications 8 ou 9, dans lequel le second élément de commande (212) de l'ensemble de poignée coulisse le long de l'axe longitudinal pour ouvrir et fermer l'élément de soupape autour de l'arbre de l'ensemble d'attache.
